# DEMANDE DE BREVET EUROPEEN

(11) **EP 0 816 488 A1**
(43) Date de publication de la demande: **07.01.1998**
(21) Numéro de dépôt: 96201763.8
(22) Date de dépôt: 25.06.1996
(51) Int. Cl.: C12N 5/04, C12M 3/06, A01H 4/00

(54) **Procédé de conditionnement de tissus de végétaux cultivés in-vitro**

(71) Demandeur: SOCIETE DES PRODUITS NESTLE S.A., 1800 Vevey (CH)
(72) Inventeur: Florin, Bruno, 37540 Saint Cyr sur Loire (FR); Ducos, Jean Paul, 37100 Tours (FR); Petiard, Vincent, 37100 Tours (FR)

(57) **Abrégé**

Procédé de conditionnement de tissus de végétaux cultivés *in-vitro* dans lequel, dans un récipient stérile, on dispose des tissus de végétaux cultivés *in-vitro* entre les faces de deux supports solides imprégnés de milieu nutritif liquide, lesdits tissus de végétaux étant maintenus solidaires avec au moins une des faces des supports par capillarité, au moins une des autres faces des supports étant également maintenue solidaire avec une des parois du récipient par capillarité, et on conserve les tissus en l'état. L'invention concerne aussi un dispositif de conditionnement destiné à mettre en oeuvre le procédé.

## Description

L'invention se rapporte à un nouveau procédé de conditionnement de tissus de végétaux cultivés *in-vitro* permettant d'améliorer la conservation desdits tissus et qui est particulièrement adapté au transport de matières végétales.

### Etat de la technique

Le transport de tissus de végétaux cultivés *in-vitro* nécessite un conditionnement adapté. Par exemple, on peut conditionner des tissus de végétaux en les plaçant dans un milieu de culture liquide ou sur un milieu semi-solide gélosé, ces milieux étant contenus dans des récipients usuels pour la culture *in-vitro*, tels que des boites de Petri ou des bouteilles. Malheureusement, ce type de conditionnement ne peut répondre aux aléas que peuvent subir un colis lors d'un transport, à savoir (1) une asphyxie des tissus par manque d'agitation dans le cas des cultures liquides; (2) un recouvrement des tissus par la gélose, dû à de trop grandes secousses, qui feront que les tissus subiront une asphyxie; (3) des contaminations résultant du contact des milieux de culture avec l'extérieur des récipients; (4) et une croissance trop importante des tissus dans le cas de certaines espèces.

On peut aussi conditionner des tissus de végétaux cultivés *in-vitro* sous une couche d'huile à des températures de réfrigération. EP408922 (Société des Produits Nestlé) décrit en effet un procédé dans lequel des embryons sont maintenus en hypoxie par immersion sous une couche d'huile, puis refroidis et conservés à une température supérieure au seuil de sensibilité au froid des embryons considérés. Bien que les embryons ainsi conservés présentent une viabilité correcte après quelque jours de conservation, cette méthode est encore sujette aux aléas des changement de températures de réfrigération lors d'un transport.

On peut aussi transporter des tissus de végétaux cultivés *in-vitro* lorsque ceux-ci sont enrobés avec un mélange d'alginate et de saccharose (Hasan et al., Plant Genetic Resources Newsletter, 103, 32-35, 1995)

On peut aussi congeler des tissus de végétaux cultivés *in-vitro*. A titre d'exemple, Chen et al. proposent de cultiver des cals en présence de sorbitol (agent déshydratant) et de diméthylsulfoxyde (agent cryoprotecteur), de les congeler lentement à - 40°C puis dans l'azote liquide (Plant Physiol., 75, 726-731, 1984). De même, Delvallée et al. proposent de cultiver des embryons zygotiques immatures en présence de saccharose (agent déshydratant) et de diméthylsulfoxyde, puis de les congeler dans l'azote liquide (Plant Science, 60, 129-136, 1989). Enfin, EP399158 (Société des Produits Nestlé) décrit un procédé dans lequel, on prétraite des embryons somatiques sur un milieu comprenant un agent de pression osmotique, puis on les refroidit et on les congèle à une température comprise entre -15°C et -40°C. Malheureusement, la congélation n'est pas toujours adaptée au transport de tissus de végétaux du fait des aléas de températures pouvant conduire à la mort des tissus.

Par ailleurs dans un domaine technique différent, on connaît des méthodes de conservation de semences ou de boutures de plantes. EP66581 (Piquepaille et al.) propose, par exemple, un dispositif pour faire germer des semences ou pour conserver des boutures de plantes, qui est constitué d'un récipient dans lequel le matériel biologique est disposé entre un premier support adhésif et un deuxième support souple pouvant être imprégné d'éléments liquides, les deux supports étant maintenus solidaires par la face adhésive du premier support, et les deux supports étant maintenus aux parois du récipient au moyen d'adhésifs. On peut noter que ce dispositif n'est pas stérilisable du fait de la présence d'adhésifs. De plus, la face adhésive du premier support est susceptible d'endommager des tissus mous, comme des tissus de végétaux cultivés *in-vitro*, ce qui limite d'autant plus l'attrait de ce dispositif dans le cadre de la présente invention.

A ce jour, on ne connaît donc pas de dispositif de conditionnement de tissus de végétaux cultivés *in-vitro*, qui soit à la fois simple à mettre en oeuvre et qui puisse protéger d'une manière satisfaisante lesdits tissus de végétaux. La présente invention se propose ainsi de fournir un nouveau procédé très simple de conditionnement de tissus de végétaux cultivés *in-vitro*, particulièrement adapté aux transports d'embryons somatiques, et qui ne nécessite que très peu de moyens techniques et de temps de mise en oeuvre.

### Résumé de l'invention

A cet effet, l'invention concerne un nouveau procédé de conditionnement de tissus de végétaux cultivés *in-vitro* dans lequel, dans a récipient stérile, on dispose des tissus de végétaux cultivés *in-vitro* entre les faces de deux supports solides imprégnés de milieu nutritif liquide, lesdits tissus de végétaux étant maintenus solidaires avec au moins une des faces des supports par capillarité, au moins une des autres faces des supports étant également maintenu solidaire avec une des parois du récipient par capillarité, et on conserve les tissus en l'état.

L'invention concerne aussi un dispositif conditionneur comprenant des tissus de végétaux cultivés *in-vitro*, ledit dispositif étant constitué d'un récipient stérile scéllable dans lequel sont disposés des tissus de végétaux cultivés *in-vitro* entre les faces de deux supports solides imprégnés de milieu nutritif liquide, lesdits tissus de végétaux état maintenus solidaires avec au moins une des faces des supports par capillarité, au moins une des autres faces des supports étant également maintenu solidaire avec une des parois du récipient par capillarité.

Bien que l'art antérieur préconise des techniques bien plus sophistiquées, on a constaté que les tissus de végétaux cultivés *in-vitro* survivent parfaitement dans les conditions de conditionnement selon l'invention pendant le temps d'un transport pouvant aller de 1 à 100 jours. Le présent procédé évite ainsi les inconvénients de l'art antérieur, car il est facile à mettre en oeuvre, les risques de contamination sont réduits, les tissus ne risquent pas d'être immergés totalement dans du milieu nutritif les tissus sont protégés des chocs grâce à leur insertion entre les supports, et les tissus sont très facilement mis en culture après conservation, car il suffit de les placer dans un milieu nutritif liquide ou semi-solide gélosé, par exemple.

D'une façon étonnante, on a pu constaté que la plupart des espèces végétales à croissance lente, comme les ligneux par exemple, ont une croissance inhibée réversiblement par de telles conditions de conditionnement. Par contre, si on veut limiter la croissance d'espèces végétales ayant une croissance plus rapide, comme les légumes, on peut aussi les déshydrater avant de les conditionner, par exemple.

Avec surprise, on a constaté que les conditions de conditionnement selon l'invention pouvaient même améliorer la capacité des embryons somatiques à se développer en plantule.

### Description détaillée de l'invention

Pour mettre en oeuvre la présent invention, on utilise donc des tissus de végétaux issus d'une culture *in-vitro*, à savoir des cals, des bourgeons qui peuvent s'être développés sur un cal, des embryons somatiques, et des racines, par exemple. L'invention se destine cependant de préférence à la conservation d'embryons somatiques de végétaux, qui sont connus pour être particulièrement sensibles à leur environnement.

Le récipient que l'on utilise peut être tout réceptacle que l'on peut stériliser, et qui peut être scellé réversiblement, par exemple. Un récipient connu de l'homme du métier et qui est disponible en grande quantité, est la boîte de Petri, que l'on peut sceller au moyen d'un film plastique, de cire ou d'une autre matière appropriée, connue de l'homme du métier, par exemple.

Les supports solides utilisés sont de préférence constitués d'une matière souple. qui peut éventuellement, mais pas nécessairement, épouser la forme d'un amas de tissus de végétaux, et qui ne se désagrège pas au cours du temps. A cet effet, cette matière peut être fibreuse en partie ou totalement, et inclure une matière capable de retenir a liquide, lesdites fibres pouvant également remplir cette fonction, par exemple. Ces supports peuvent ainsi être constitués, seul ou en combinaison, de cellulose (papier buvard), de silice, de verte, de polymères de dextrane ou de chitine, de protéines comme la soie, ou de polymères synthétiques du type polystyrène, polyacrylate, polyméthacrylate, polyvinyle, et polyamide comme le nylon, par exemple. De plus, la matière retenant l'eau et pouvant être inclue aux fibres peut être constituée de composés organiques ou inorganiques qui absorbent les liquides aqueux pour former un hydrogel, comme par exemple des polyacrylamides ou des polysaccharides comme la gélose. Cependant, cette matière ne doit pas pouvoir s'écouler du support.

Les supports doivent pouvoir être imprégnés d'un liquide comprenant les éléments nutritifs essentiels à la survie des tissus. Parmi, les milieux nutritifs connus de l'homme du métier, on peut citer à titre d'exemple le milieu de Murashige et Skoog complété des vitamines de Skoog ou de Linsmayer (Physiol. Plant., 1962, vol. 15, p. 473; Physiol. Plant., 1965, vol 18, p. 100).

De préférence, au moins un des supports peut être imprégné de liquide nutritif jusqu'au seuil limite de rétention d'eau du support après lequel celui-ci ne peut plus retenir de liquide. La quantité de liquide imprégnée est de préférence telle qu'elle assure une humidité relative du récipient d'au moins 95-100%, de préférence au moins 98%, pendant au moins 1 à 5 jours. Le fait de disposer les tissus de végétaux entre un support sec et un deuxième support imprégné permet d'obtenir une lente déshydratation contrôlée des tissus, ledit support sec s'imprégnant en effet lentement de liquide qui migre du support imprégné vers le support sec.

Il est avantageux d'utiliser des supports de faible épaisseur et de poids, de façon à ne pas endommager les tissus, et de façon aussi à favoriser une bonne diffusion de l'oxygène, ainsi qu'une adhérence par capillarité des supports entre eux et à la surface du récipient. A titre d'exemple, on peut utiliser des filtres ayant 0,03 à 1 g, par exemple 0,03 à 0,1g, et capable d'absorber plus de 2 à 10 fois leur poids initial de liquide nutritif, par exemple.

On peut disposer entre les supports les tissus de végétaux en amas ou individuellement, sans que la viabilité des tissus en soit affectée. Avant de disposer les tissus, il n'est pas nécessaire de séparer le milieu de culture résiduel des tissus de végétaux prélevés de leur milieu de culture, par exemple par aspiration par le vide au travers d'un filtre. Cependant, si l'on veut assurer une déshydratation plus prononcée des tissus, comme décrit ci-dessus, on peut aussi séparer le milieu de culture résiduel. Finalement, on peut disposer 1 à 100 tissus de végétaux cultivés *in-vitro* par cm² de support, sachant que les tissus peuvent être de 0,05 à 1 cm de long, et par exemple de 0,05 à 0,3 cm de long dans le cas d'embryons somatiques pris au stade de développement "torpille".

Il peut être souhaitable de s'assurer que les supports sont aussi bien maintenus solidaires par capillarité. Pour cela, on peut prévoir sur les supports, à la périphérie de la zone où l'on dispose les tissus de végétaux, une zone circulaire autour des tissus, suffisamment large, de l'ordre d'au moins 1 cm, qui restera vierge de tous tissus et qui est destinée à assurer une bonne adhérence des supports entre eux.

Enfin les deux supports, comprenant inclus les tissus de végétaux cultivés *in-vitro*, sont déposés dans le récipient approprié, le maintien d'au moins un des supports se faisant simplement par capillarité d'une surface d'un des supports à une des surfaces du récipient.

Le présent procédé peut être appliqué à des objets de petite taille, il permet un transport aisé de grands effectifs, et est donc compatible avec une diffusion commerciale. Par exemple, si l'on veut diffuser une grande quantité d'embryons somatiques d'une espèce végétale donnée, on peut disposer selon le présent procédé dans des boites de Petri standard de 5,5 cm de diamètre et de 1 cm de hauteur, environ 100 embryons ayant 0,05 à 0,2 cm de longueur. Un colis de 1 m3 contiendra ainsi 3280 boites, soit environ 328000 embryons, par exemple. Pour comparaison, un transport de plantules feuillées avec racines et paire de feuilles comprend généralement 5000 à 10000 plantules par m3, par exemple

Le présent procédé s'applique à toutes espèces végétales, notamment les plantes ligneuses comme le caféier, le cacaoyer, l'hévéa, le bananier, le cocotier et le palmier à huile, et notamment les légumes comme la tomate, la carotte, le petit pois, le céleri et le fenouil. L'homme du métier dispose de nombreuses techniques pour obtenir des tissus de végétaux cultivées *in-vitro*, et peut même les adapter à chaque espèce végétale pouvant requérir des conditions particulières quant à l'obtention de certains types de tissus, comme par exemple les embryons, les bourgeons ou les racines. A titre d'indication, l'embryogenèse somatique du cacaoyer, du caféier, du palmier à huile, de la carotte peut être réalisée selon les protocoles décrits respectivement dans US5312801, par Zamarripa (Café Cacao et Thé, 35, 233-244, 1991), par Engelmann et al. (Oléagineux, 41, 169-172, 1986) et dans EP399158.

Le présent procédé s'applique en particulier à toutes les espèces végétales, notamment les plantes ligneuses, présentant en culture *in-vitro* une croissance lente caractérisée par un taux de multiplication de moins de 10 par semaine, c'est à dire moins de 10 fois plus de matière fraîche chaque semaine, par exemple. A titre d'indication, un embryon ayant une croissance lente passe d'une taille de 1 mm à 10 mm en 1 à 2 mois (la plus grande longueur est considérée), par exemple.

On conserve les tissus de végétaux à croissance lente pendant le temps d'un transport (1-100 jours) de préférence à l'obscurité et/ou à une température de 10°C à 35°C, en particulier 15-30°C.

Si les tissus ont été déshydratés, il est aussi possible de conserver les tissus selon l'invention à l'obscurité et/ou à des températures de réfrigération, voire même de congélation (par exemple -4°C) jusqu'à 30-35°C. Toutefois, on prendra garde à ne pas déshydrater des tissus comprenant de la chlorophylle, car un tel traitement est alors susceptible d'endommager irrémédiablement les tissus.

Pour déshydrater les tissus, en outre voire en addition de la technique décrite cidessus, il suffit par exemple de cultiver les tissus sur un milieu de culture traditionnel, auquel on ajoute un agent de pression osmotique et auquel on peut également rajouter certaines substances organiques, telles que la vitamine B1, l'acide nicotinique ou l'adénine. Cet agent peut être choisi par l'homme du métier parmi les substances susceptibles de pénétrer les tissus et d'assurer une bonne pression osmotique afin d'obtenir une déshydratation correcte de la cellule, sans en influencer la perméabilité membranaire. Cet agent peut être un sucre, tel que le saccharose ou le tréhalose, ou toute autre substance connue pour remplir les mêmes fonctions. La concentration en agent de pression osmotique du milieu de culture n'est de préférence pas trop faible afin d'assurer une déshydratation correcte de la cellule. Elle ne doit pas être non plus trop forte afin de ne pas détériorer les tissus ou empêcher leur reprise de croissance après conservation. A titre d'indication, on peut déshydrater des tissus sur un milieu de culture comprenant 50 à 190 g/l de saccharose, en particulier 100 à 150 g/l.

La déshydratation des tissus peut être aussi un moyen pour inhiber réversiblement la croissance des tissus, lorsqu'il apparaît que le procédé selon l'invention n'est pas suffisant pour inhiber suffisamment la croissance desdits tissus A cet effet, les tissus ayant un taux de multiplication supérieur à 10 par semaine (10 fois plus de matière fraîche) sont de préférence déshydratés comme décrit ci-dessus, par exemple. A titre d'indication, un embryon ayant une croissance rapide passe d'une taille de 1 mm à 10 mm en 4 à 6 jours (la plus grande longueur est considérée), par exemple. Parmi les végétaux ayant une croissance rapide en culture *in-vitro*, on peut compter la carotte, le céleri et le fenouil, par exemple.

Après transport, le déconditionnement des tissus est très facile, dans le mesure où il suffit de secouer les supports au dessus d'un milieu de culture pour en détacher les tissus. On peut aussi les repiquer individuellement avec des ustensiles connues de l'homme du métier.

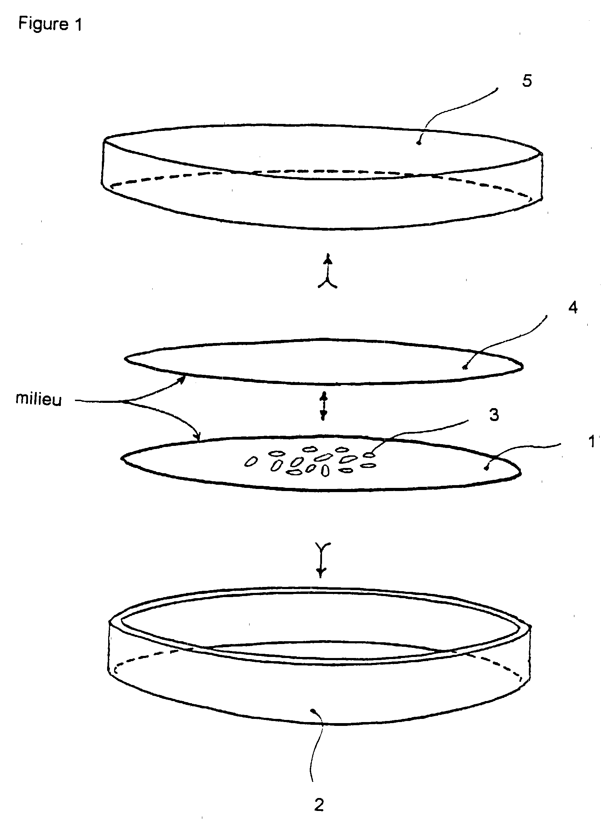

L'invention est décrite plus en détail dans les exemples d'application présentés ci-après, et en référence à l'unique figure 1 qui représente schématiquement un dispositif selon l'invention. Il va de soi que les caractéristiques du procédé selon l'invention ne sont pas limités aux exemples particuliers décrits ci-après, qui peuvent être adaptées par l'homme du métier à toutes espèces et tissus de végétaux.

Dans la figure 1, la face d'un support 1(filtre en fibres de verte), imprégné de liquide nutritif, est réversiblement fixée par capillarité dans le fond 2 d'une boîte de Petri, l'autre face dudit support recevant des tissus de végétaux cultivés *in-vitro* 3. Un deuxième support 4 (filtre en fibres de verte), pouvant être imprégné de liquide nutritif, vient au contact des tissus 3 et du premier support 2. Enfin, le couvercle 5 de la boîte de Petri permet de fermer le récipient, un scellage définitif pouvant être réalisée au moyen d'un film plastique, par exemple.

### Exemple 1

On dépose, un par un, 100 embryons somatiques de caféier Robusta (*Coffea canephora*), obtenus au stade torpille selon le protocole de Zamarripa et al. (Café Cacao et Thé 35, 233-244, 1991), entre deux filtres de fibre de verte (Whatman GFC, diamètre 5 cm, 0,01g de poids sec) imprégnés du milieu de culture liquide de Murashige et Skoog. On peut noter que les filtres imprégnés ne relachent pas de liquide. On dispose les filtres dans une boîte de Petri.

On prépare, de la même façon, plusieurs boîte de Petri comprenant des embryons de caféier, on les conserve à 20°C, puis périodiquement on remet les embryons d'une boîte en culture sur un milieu semi-solide, et on détermine le nombre d'embryons survivant (viabilité) et le nombre d'embryons capable de se développer en une plantule (conversion).

Les résultats, présentés dans le tableau ci-après, montrent que tous les embryons survivent parfaitement à 15 jours de conservation, et que la conversion en plantules d'embryons conservés pendant 1 à 30 jours est bien meilleure que celle d'embryons pris au stade torpille et n'ayant pas subi de conditionnement.

| Nombre de jours | Viabilité % | Conversion (%) |
|---|---|---|
| 0 (contrôle) | 97 ± 3 | 32 ± 4 |
| 15 | 100 | 71 ± 5 |
| 30 | 89 ± 11 | 52 ± 3 |
| 45 | 73 ± 13 | 34 ± 9 |
| 60 | 36 ± 31 | 12 ± 10 |

### Exemple 2

On dépose, en amas, 1 g d'embryons somatiques de caféier Robusta (*Coffea canephora*), obtenus au stade torpille selon le protocole de Zamarripa et al., entre deux filtre de verte (Whatman GFC, diamètre 5 cm, 0,01g de poids sec) imprégnés du milieu de culture liquide de Murashige et Skoog. On peut noter que les filtres imprégnés ne relâchent pas de liquide. On dispose alors les filtres dans une boîte de Petri. On prépare, de la même façon, plusieurs boîte de Petri comprenant des embryons de caféier, on les conserve à 20°C pendant 4 semaines, puis on remet 2450 embryons sur un milieu semi-solide, et on détermine le nombre d'embryons capable de se développer en une plantule (conversion). Les résultats montrent que 794 plantules se développent en 5 mois. Les embryons conditionnés présentent donc un taux de conversion de 32%, ce qui est similaire aux taux de conversion présentés traditionnellement par des embryons de café non-conditionnés (voir le tableau).

### Exemple 3

D'une façon similaire à l'exemple 2, on conditionne 0,1 g de cals de caféier Robusta (*Coffea canephora*) par boîte de Petri, on conserve les cals pendant 1 mois à 20 °C, on les remet en culture sur un milieu semi-solide pendant 1 mois, puis on détermine le poids de matériel végétal ainsi obtenu (0,55 ± 0,23 g de matière fraîche) que l'on compare au poids de matériel végétal d'une culture témoin non conditionnée de 1 mois qui a été ensemencée avec 0,1 g de cals frais (0,59 ± 0,18 g de matière fraîche). Les résultats montrent ainsi que les cals de caféier survivent parfaitement au conditionnement.

### Exemple 4

On prétraite des embryons somatiques pris au stade torpille sur un milieu comprenant un agent de pression osmotique, comme décrit dans EP399158, puis on les conditionne comme décrit à l'exemple 2. Les embryons de carotte ainsi conditionnés pendant plusieurs jours conservent une viabilité et une capacité de se développer en une plantule, qui sont comparables à des embryons de carotte non-conditionnés.

### Exemple 5

On conditionne des embryons somatiques de carotte pris au stade torpille, comme décrit à l'exemple 2, à la différence près que l'on déshydrate préalablement les embryons sur un milieu comprenant 100 g/l de saccharose, puis on les dispose entre un filtre de fibre de verte imprégné de liquide nutritif et un autre filtre sec. Les embryons de carotte ainsi conditionnés pendant plusieurs jours conservent une viabilité et une capacité de se développer en une plantule, qui sont comparables à des embryons de carotte non-conditionnés.

## Revendications

1. Procédé de conditionnement de tissus de végétaux cultivés *in-vitro* dans lequel, dans un récipient stérile, on dispose des tissus de végétaux cultivés *in-vitro* entre les faces de deux supports solides imprégnés de milieu nutritif liquide, lesdits tissus de végétaux étant maintenus solidaires avec au moins une des faces des supports par capillarité, au moins une des autres faces des supports étant également maintenue solidaire avec une des parois du récipient par capillarité, et on conserve les tissus.

2. Procédé selon la revendication 1 dans lequel, les supports sont constitués, en partie ou totalement, d'une matière fibreuse incluant ou non une matière capable de retenir a liquide.

3. Procédé selon la revendication 1 dans lequel, la quantité de liquide imprégnée dans au moins un des supports permet d'assurer une humidité relative du récipient d'au moins 95% pendant au moins 1 à 5 jours.

4. Procédé selon la revendication 1 dans lequel, on utilise des embryons ayant un taux de multiplication de moins de 10 par semaine.

5. Procédé selon la revendication 1 dans lequel, un des deux supports est au départ sec.

6. Procédé selon l'une des revendications 1 à 5 dans lequel, on utilise des embryons déshydratés.

7. Procédé selon au moins l'une des revendication 5 et 6 dans lequel, on utilise des embryons ayant un taux de multiplication de plus de 10 par semaine.

8. Procédé selon la revendication 7 dans lequel, on conserve les embryons de -4°C à 35°C.

9. Dispositif conditionneur comprenant des tissus de végétaux cultivés *in-vitro*, ledit dispositif étant constitué d'un récipient stérile scéllable dans lequel sont disposés des tissus de végétaux cultivés *in-vitro* entre les faces de deux supports solides imprégnés de milieu nutritif liquide, lesdits tissus de végétaux étant maintenus solidaires avec au moins une des faces des supports par capillarité, au moins une des autres faces des supports étant également maintenue solidaire avec une des parois du récipient par capillarité.
